(19) Europäisches Patentamt
European Patent Office
Office européen des brevets

(11) **EP 1 811 293 B1**

(12) **EUROPÄISCHE PATENTSCHRIFT**

(45) Veröffentlichungstag und Bekanntmachung des Hinweises auf die Patenterteilung:
**26.09.2018 Patentblatt 2018/39**

(51) Int Cl.:
*A01D 41/12* (2006.01)   *A01D 41/127* (2006.01)
*G01N 33/02* (2006.01)   *G01N 33/10* (2006.01)
*G01N 27/22* (2006.01)

(21) Anmeldenummer: **07105930.7**

(22) Anmeldetag: **22.10.2002**

(54) **Kornfeuchtigkeitssensor**

Grain Moisture Sensor

Capteur d'humidité de grain

(84) Benannte Vertragsstaaten:
**BE DE DK FR IT**

(30) Priorität: **25.10.2001 US 3884**

(43) Veröffentlichungstag der Anmeldung:
**25.07.2007 Patentblatt 2007/30**

(62) Dokumentnummer(n) der früheren Anmeldung(en) nach Art. 76 EPÜ:
**04100529.9 / 1 421 841**
**02102471.6 / 1 305 994**

(73) Patentinhaber: **Deere & Company**
**Moline, IL 61265 (US)**

(72) Erfinder:
• **Rains, Gerald, E.**
**Bettendorf, IL 52722 (US)**
• **Phelan, James, Joseph**
**Bettendorf, IA 52722 (US)**
• **Slavens, Zachary W.**
**Bettendorf, IA 52722 (US)**
• **Kozicki, Andrzej**
**Milan, IL 61264 (US)**
• **Funk, Robert, C.**
**Auburn, IL 62615 (US)**

(74) Vertreter: **Holst, Sönke**
**John Deere GmbH & Co. KG**
**Global Intellectual Property Services**
**John-Deere-Strasse 70**
**68163 Mannheim (DE)**

(56) Entgegenhaltungen:
EP-A1- 0 908 086   GB-A- 2 210 693
US-A- 4 399 404   US-B1- 6 285 198

• K.C. LAWRENCE ET AL.: "Wheat Moisture Determination by 1- to 110-MHz Swept-Frequency Admittance Measurements" TRANSACTIONS OF THE ASAE, Bd. 41, Nr. 1, Februar 1998 (1998-02), Seiten 135-142, XP009085464

**Beschreibung**

Die Erfindung betrifft einen Kornfeuchtigkeitssensor für einen Mähdrescher.

Kornfeuchtigkeitssensoren werden in Mähdreschern verwendet, insbesondere in Präzisionslandwirtschaftsanwendungen. Kontinuierliche oder momentane Kornfeuchtigkeitsmessungen erlauben einem Bediener, die Feuchtigkeit des Korns während des Erntens zu beobachten. In Verbindung mit einer Einrichtung zur Ortsbestimmung, z. B. GPS, kann ein Feuchtigkeitssensor zur Feuchtigkeitskartierung verwendet werden. Außerdem werden Feuchtigkeitssensoren in Ertragskartierungsanwendungen genutzt. Wenn sie in Kombination mit einem Kornflusssensor benutzt werden, wird die Information des Feuchtigkeitssensors häufig benutzt, die Menge trockenen Getreidevolumens auf einem Feld und das Getreidevolumen auf einer Flächeneinheit basierend auf der Menge feuchten Getreides und des Feuchtigkeitsgehaltes zu berechnen.

Das Dokument "Wheat Moisture Determination by 1- to 110-MHz Swept-Frequency Admittance Measurements" von K. C. Lawrence et al., Transactions of the ASAE, Bd. 41, Nr. 1, Februar 1998, Seiten 135 bis 142, beschreibt eine Anordnung zur Messung der Kornfeuchtigkeit mit einem Plattenkondensator, in den eine Kornprobe eingefüllt wird. Es werden bei unterschiedlichen Frequenzen mittels eines Impedanzanalysators jeweils die komplexen Leitwerte des leeren Plattenkondensators und des mit Korn gefüllten Plattenkondensators gemessen. Anhand der gemessenen komplexen Leitwerte werden die Kapazitäten und die Effektivwerte der Leitwerte berechnet, anhand der die Kornfeuchte berechnet wird.

Die GB 2 210 693 A und die US 4 399 404 A beschreiben Feuchtigkeitsmessgeräte für Korn, bei der ein Messsignal einem als Messzelle dienenden Kondensator und einem Referenzkondensator zugeführt wird.

Die der Erfindung zu Grunde liegende Aufgabe wird darin gesehen, einen verbesserten Kornfeuchtigkeitssensor für einen Mähdrescher für die oben genannten Anwendungen bereitzustellen.

Diese Aufgabe wird erfindungsgemäß durch die Lehre des Patentanspruches 1 gelöst, wobei in den weiteren Patentansprüchen Merkmale aufgeführt sind, die die Lösung in vorteilhafter Weise weiterentwickeln.

Der erfindungsgemäße Kornfeuchtigkeitssensor umfasst eine Zelle zur Aufnahme von Korn, die eine elektrisch beaufschlagte Platte umfasst, an die Anregungsspannungen angelegt werden und eine Sensorplatte in der Nähe zur elektrisch beaufschlagten Platte, die sich parallel dazu erstreckt, um den durch die Zelle fließenden Strom zu messen. Die vorliegende Erfindung ermöglicht eine Messung der komplexen Dielektrizitätskonstante des Korns. Es wird außerdem ermöglicht, diese bei unterschiedlichen Frequenzen zu messen. Ein Schaltkreis misst sowohl die Real- als auch die Imaginärteile sowohl der Anregungsspannung als auch des Stroms in der Sensorplatte. Aus diesen Werten wird die komplexe Dielektrizitätskonstante der Zelle (oder ein damit im Zusammenhang stehender Wert) ermittelt. Anstelle einer leeren Zelle werden kalibrierte Referenzleitwerte verwendet, um die komplexe Dielektrizitätskonstante des Korns zu errechnen. Der Schaltkreis ist eingerichtet, Messungen für den Leitwert einer der Referenzen und den Leitwert der leeren Zelle bei denselben Umgebungsbedingungen durchzuführen, um zu Kalibrierzwecken einen Referenzleitwert anstelle eines Leitwerts der leeren Zelle zu verwenden, wobei der Referenzleitwert über einen Kalibrierfaktor F, der das Verhältnis von Leitwert der Referenz zum Leitwert der leeren Zelle wiedergibt, bestimmt wird, und er ist eingerichtet, unter mehreren Referenzen auszuwählen. Vorzugsweise ist der Sensorplatte und auch der Füllstandssensorplatte eine Abschirmung zugeordnet. Die Sensorplatte ist zwischen der Abschirmung und der elektrisch beaufschlagten Platte angeordnet. Die Abschirmung ist elektrisch von der Sensorplatte isoliert, befindet sich aber auf demselben Potential. Die Abschirmung ist parallel zur Sensorplatte orientiert und von größeren Abmessungen als die Sensorplatte, erstreckt sich somit über ihre Ränder hinaus, um das elektrische Feld zu formen. Das Vorhandensein der Abschirmung stellt den Vorteil gerader elektrischer Feldlinien bereit, die senkrecht zur Sensorplatte verlaufen und über das gesamte Volumen zwischen den parallelen Platten eine gleichförmige Dichte aufweisen. Das hat verminderte

Randeffekte und eine gleichförmige elektrische Felddichte zur vorteilhaften Folge, so dass über die gesamte Zelle eine konstante Empfindlichkeit erreicht wird. Außerdem schirmt die Abschirmung die Sensorplatte von externen elektrischen Feldern ab, die von anderen Feldern als der elektrisch beaufschlagten Platte erzeugt werden.

Zweckmäßigerweise ist ein Temperatursensor vorgesehen, der die Temperatur des Korns in der Zelle zumindest näherungsweise erfasst. Sein Messwert wird bei der Massenberechnung herangezogen.

Zur Messung der Real- und Imaginärteile der Spannung und des Stroms können Mischer verwendet werden. Dieses synchrone Detektionsverfahren, wie es auch an Lock-in-Verstärkern benutzt wird, hat einen sehr schmalbandigen Filtereffekt, der den Störgeräuscheinfluss auf die Messung wesentlich reduziert. Zur Messung des Stroms an der Sensorplatte wird ein Verfahren zur Messung niedriger Ströme mit virtuellem Erdpotential genutzt, um die Vorteile einer wesentlichen Verminderung des Einflusses parasitärer Elemente am Strommessknoten zu erzielen.

Der Kornfeuchtigkeitssensor umfasst vorzugsweise eine Füllstandssensorplatte nahe der Sensorplatte, um festzustellen, wenn die Zelle gefüllt ist. Die Füllstandssensorplatte befindet sich in der Regel an der Einlassseite der Zelle. Der sie durchfließende Strom gibt einen Hinweis auf den Füllstand der Zelle: wenn die auf die Fläche bezogene Stromdichte an der Füllstandssensorplatte der Stromdichte an der Sensorplatte entspricht, ist die Zelle gefüllt.

In den Zeichnungen ist ein nachfolgend näher beschriebenes Ausführungsbeispiel der Erfindung dargestellt.

Es zeigt:

Fig. 1A          eine Seitenansicht eines Mähdreschers mit einem erfindungsgemäßen Kornfeuchtigkeitssensor,

Fig. 1B          eine Seitenansicht eines erfindungsgemäßen Kornfeuchtigkeitssensors, der an einem Mähdrescher angebracht und in einer Füllposition ist,

Fig. 1C          eine Seitenansicht eines erfindungsgemäßen Kornfeuchtigkeitssensors, der an einem Mähdrescher angebracht und in einer Messposition ist,

Fig. 2           ein seitlicher Querschnitt durch die Zelle eines erfindungsgemäßen Kornfeuchtigkeitssensors,

Fig. 3           ein seitlicher Querschnitt durch die Zelle aus Figur 2, in der die Äquipotentiallinien des elektrischen Feldes dargestellt sind, das entsteht, wenn eine Anregungsspannung an die elektrisch beaufschlagte Platte angelegt wird,

Fig. 4           ein schematisches Schaltdiagramm eines Modells der erfindungsgemäßen Zelle,

Fig. 5           ein Blockdiagramm des Admittanzmeßkreises eines erfindungsgemäßen Kornfeuchtesensors, und

Fig. 6A und 6B     Blockdiagramme eines erfindungsgemäßen Feuchtesensorschaltkreises.

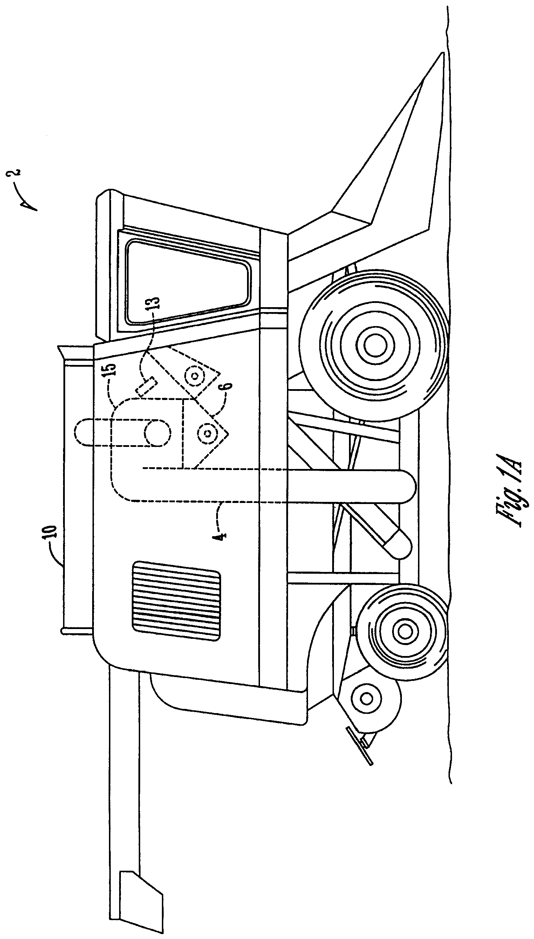

Die Figur 1A zeigt einen Mähdrescher mit einem erfindungsgemäßen Kornfeuchtigkeitssensor. In der Figur 1A ist der Mähdrescher 2 mit einem Korntank 10 dargestellt. Außerdem wird ein Elevator 4 für sauberes Korn gezeigt. Korn aus dem Elevator 4 für sauberes Korn bewegt sich zum Übergangsgehäuse 15 des Korntanks 10. Der Sumpf 6 des Übergangsgehäuses 15 wird auch gezeigt.

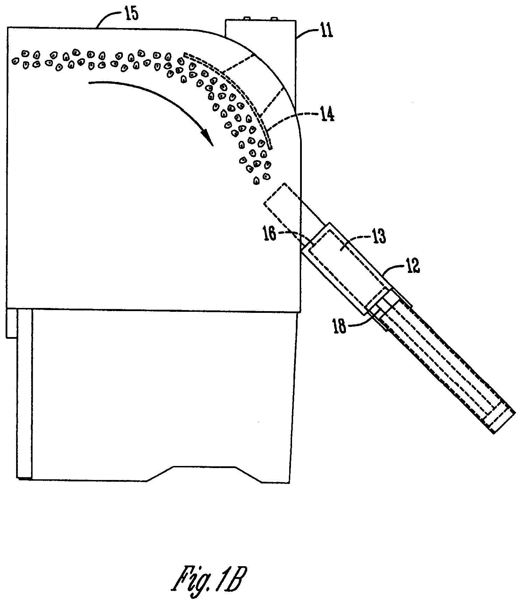

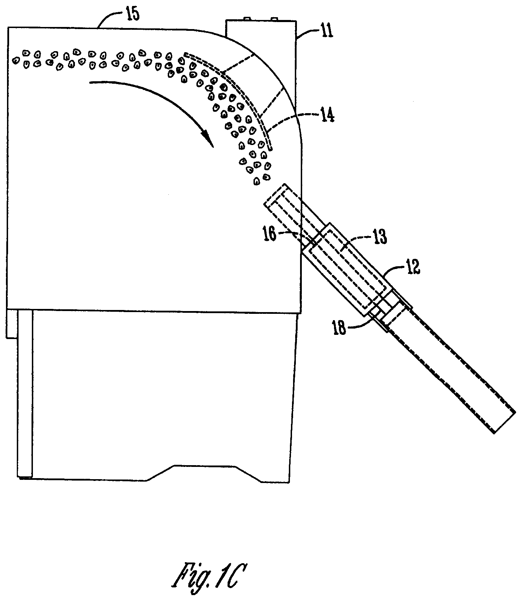

Die Figuren 1B und 1C zeigen Seitenansichten eines erfindungsgemäßen Kornfeuchtigkeitssensors, der an einem Mähdrescher angebracht ist. Der dargestellte Korntank 10 umfasst den Kornfeuchtigkeitssensor 12. Der Kornfeuchtigkeitssensor 12 ist im Korntank 10 des Mähdreschers 2 nahe dem Massenflusssensor 11 angeordnet. Die Öffnung 16 der Zelle 13 ist unterhalb der Prallplatte 14 im Übergangsgehäuse 15 angeordnet. Obwohl eine Prallplatte 14 dargestellt ist, beinhaltet die vorliegende Erfindung, dass andere Deflektoren verwendet werden können. An dieser Stelle wird die Zelle 13 aufgrund der direkten oder indirekten Geschwindigkeiten des Korns aktiv gefüllt, die durch die (nicht gezeigten) Paddel des Elevators 4 für sauberes Korn erzeugt werden. Dies erlaubt, dass die Zelle 13 mit hohen Raten gefüllt wird. Dies vermindert jegliche mit langsamen Zykluszeiten verbundene Probleme, die mit Bedingungen kleiner Flüsse verbunden sind, da hier die Zelle 13 wegen ihrer Anordnung innerhalb des vom Elevator 4 für sauberes Korn erzeugten Stroms mit einer hohen Rate gefüllt wird. Die Zelle 13 ist mit ihrem Einlass ausgerichtet. Ein Stößel mit Kolben 18 mit einem elektrischen Aktor wird verwendet, die Kornprobe zurück aus der Einlassöffnung 16 zu zwingen. In der Figur 1C sind die Zelle 13 und der Stößel mit Kolben 18 in einer Messposition.

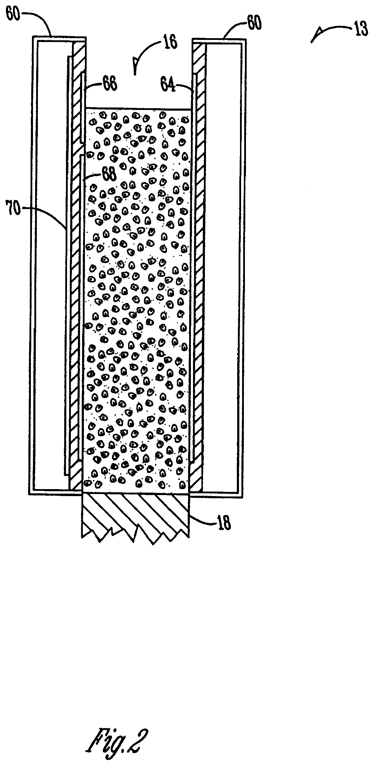

Die Figur 2 stellt eine Seitenansicht einer erfindungsgemäßen Zelle 13 dar. Die Öffnung 16 der Zelle 13 oder der Zwischenraum ist mit Korn gefüllt. An beiden Seiten des Zwischenraums sind parallele Kondensatorplatten 64 und 68. Die elektrisch beaufschlagte Platte 64 ist die Platte, an die eine Anregungsspannung angelegt ist. Die Sensorplatte 68 ist die Platte, an der der Strom gemessen wird, der die Zelle 13 durchströmt. Die Füllstandssensorplatte 66 ist nahe der Sensorplatte 68 und parallel zur elektrisch beaufschlagten Platte 64.

Die Füllstandssensorplatte 66 zur Erfassung des Füllstands der Zelle 13 ist von einem Viertel der Größe der Sensorplatte 68. Um festzustellen, ob oder wann die Zelle 13 gefüllt ist, sollte die Füllstandssensorplatte 66 einen Messwert bereitstellen, der ein Viertel des Messwerts der Sensorplatte 68 ist. Obwohl in dieser Ausführungsform die Füllstandssensorplatte 66 ein Viertel der Größe der Sensorplatte 68 hat, beinhaltet die vorliegende Erfindung verschiedene Änderungen in den Größen der Platten 64, 66 und 68. Dies ist nur ein Beispiel einer geeigneten und nützlichen relativen Größe.

Die Abschirmung 70 ist strategisch günstig hinter der Sensorplatte 68 und der Füllstandssensorplatte 66 angeordnet. Die Abschirmung 70 ist parallel zur Sensorplatte 68 und größer dimensioniert, um das elektrische Feld zu formen. Außerdem schirmt die Abschirmung 70 die Sensorplatte 68 von äußeren elektrischen Feldern ab, die von anderen Quellen als der elektrisch beaufschlagten Platte 64 erzeugt werden.

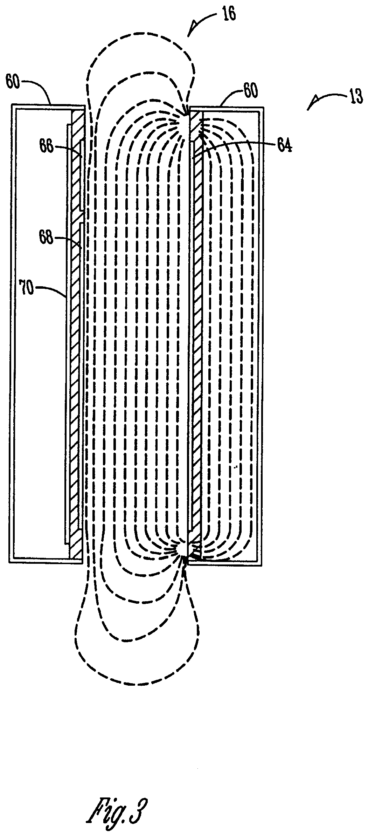

Die Figur 3 illustriert die Zelle 13 des Feuchtigkeitssensors 12 mit den Äquipotentiallinien des elektrischen Felds, das erzeugt wird, wenn ein Anregungssignal an die elektrisch beaufschlagte Platte 64 angelegt wird. Wegen der räumlichen Anordnung der Abschirmung 70, die von der Sensorplatte 68 elektrisch isoliert ist, aber auf demselben Potential liegt, ist die Wirkung auf die elektrischen Feldlinien in der Nachbarschaft der Sensorplatte 68, dass ein Äquivalent eines idealen Parallelplattenkondensators ohne Randeffekte erzeugt wird. Die elektrischen Feldlinien sind gerader Natur und senkrecht zur Sensorplatte 68 und zur Füllstandssensorplatte 66. Außerdem sind die elektrischen Feldlinien in der gesamten Region zwischen den parallelen Platten 66, 68 gleichförmiger Dichte. Das Ergebnis ist, dass Randeffekte vermindert sind. Randeffekte erzeugen unkontrollierbare Einflüsse auf die Messungen durch von Korn sich unterscheidende Materialien, die sich nahe aber außerhalb der Zelle 13 befinden. Hier zeigen die geraden elektrischen Feldlinien innerhalb der Zelle 13, dass die Zelle 13 im Wesentlichen gegen derartige Einflüsse immun ist. Außerdem stellt die gleichförmige elektrische Felddichte eine konstante Empfindlichkeit in der gesamten Zelle 13 bereit. Zusätzlich sind die ganze Zelle 13 und die Elektronik in einem Metallgehäuse 60 enthalten. Das Metallgehäuse 60 dient als Schirm gegen elektromagnetische Störungen und isoliert weiterhin die ganze Zelle 13 von anderen Quellen elektromagnetischer Energie.

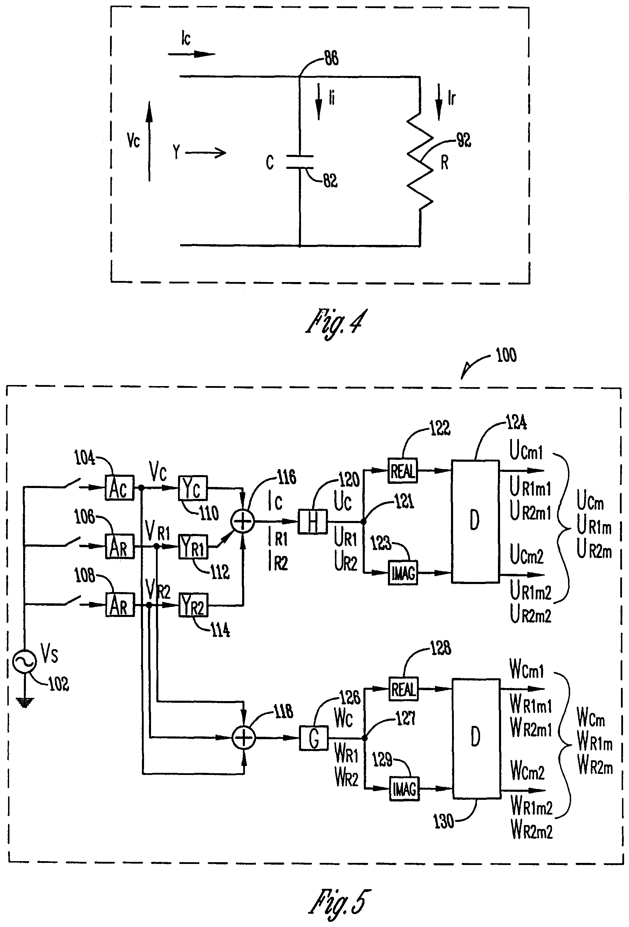

Die vorliegende Erfindung ermöglicht Kornfeuchtigkeitsmessungen basierend auf der Messung der komplexen relativen Dielektrizitätskonstante des Korns, die im Folgenden als komplexe Dielektrizitätskonstante bezeichnet wird. Die Figur 4 illustriert ein schematisches Diagramm eines Schaltkreises, der der erfindungsgemäßen Kondensatorzelle 13 elektrisch äquivalent ist. Diese Ersatzschaltung umfasst einen idealen Kondensator 82 mit einem Wert C, der parallel mit einem idealen Widerstand 92 mit dem Wert R geschaltet ist. Der ideale Kondensator 82 repräsentiert die kapazitive oder energiespeichernde Eigenschaft der Zelle 13 und der ideale Widerstand 92 repräsentiert die konduktive oder energiedissipative Eigenschaft der Zelle 13. C und R hängen von der Anregungsfrequenz und der Feuchtigkeit, Temperatur und bestimmten anderen Eigenschaften des Korns ab.

Der komplexe Leitwert der Zelle 13 ist

$$Y = (1/R) + j\omega C,$$

wobei $\omega = 2\pi f$, f die Anregungsfrequenz und j die imaginäre Einheit ist. Wenn die Zelle 13 leer ist, hat sie im Wesentlichen keine energiedissipativen Eigenschaften. Ihr Leitwert ist sehr nahe dem eines idealen Kondensators mit dem Wert $C_{CE}$:

$$Y_{CE} = j\omega C_{CE}.$$

Wenn die Zelle 13 mit Korn gefüllt ist, hat sie sowohl energiedissipative und energiespeichernde Eigenschaften. Ihr Leitwert ist

$$Y_{CF} = (1/R_{CF}) + j\omega C_{CF}.$$

Durch Teilen des Leitwerts der gefüllten Zelle 13 durch den der leeren Zelle 13 erhält man:

$$Y_{CF}/Y_{CE} = C_{CF}/C_{CE} - j/\omega C_{CE}R_{CE} == \varepsilon$$

Dieses Verhältnis ist die komplexe Dielektrizitätskonstante des Korns. Die komplexe Dielektrizitätskonstante ist eine intrinsische Materialeigenschaft und hängt nur von der Anregungsfrequenz und der Feuchtigkeit, Temperatur und bestimmter anderer Eigenschaften des Korns ab. Sie ist unabhängig von den Abmessungen und der Form der Zelle 13. Die komplexe Dielektrizitätskonstante wird im Allgemeinen folgendermaßen geschrieben:

$$\varepsilon = \varepsilon' - j\,\varepsilon'',$$

wobei $\varepsilon'$ die Dielektrizitätskonstante und $\varepsilon''$ der Verlustfaktor ist.

Es ist eine Aufgabe des Schaltkreises der vorliegenden Erfindung, den Leitwert der leeren Zelle 13 und der vollen Zelle 13 zu messen, um die obigen Gleichungen zu verwenden, um die komplexe Dielektrizitätskonstante des Korns zu berechnen. Wie in der Figur 4 gezeigt, wird die komplexe Anregungsspannung $V_C$ über den Schaltkreis gelegt. Der entstehende komplexe Strom $I_C$ fließt durch den Schaltkreis. $V_C$ hat eine reelle Komponente $V_r$ und eine imaginäre Komponente $V_i$:

$$V_C = V_r + j\,V_i$$

$I_C$ hat eine reelle Komponente $I_r$ und eine imaginäre Komponente $I_i$:

$$I_C = I_r + j \ I_i$$

Durch die Messung von $V_r$, $V_i$, $I_r$ und $I_i$ kann der komplexe Leitwert Y unter Verwendung komplexer Arithmetik berechnet werden:

$$Y = I_c/V_c = (I_r+jI_i)/(V_r+jV_i).$$

Die Figur 5 illustriert den im erfindungsgemäßen Kornfeuchtigkeitssensor verwendeten Schaltkreis zur Messung des Leitwerts. In der Figur 5 ist der Schaltkreis 100 zur Messung des Leitwerts gezeigt. Zu Erklärungszwecken werden die folgenden Definitionen verwendet:

$U_{R1m}$ wird als gemessener Wert des unten definierten $U_{R1}$ definiert.
$U_{R1m1}$ ist der Realteil (gleichphasiger Signalwert) von $U_{R1m}$.
$U_{R1m2}$ ist der Imaginärteil (Quadratur-Signalwert) von $U_{R1m}$.

$U_{R2m}$ wird als gemessener Wert des unten definierten $U_{R2}$ definiert.
$U_{R2m1}$ ist der Realteil (gleichphasiger Signalwert) von $U_{R2m}$.
$U_{R2m2}$ ist der Imaginärteil (Quadratur-Signalwert) von $U_{R2m}$.

$UC_m$ wird als gemessener Wert des unten definierten $U_C$ definiert.
$U_{Cm1}$ ist der Realteil (gleichphasiger Signalwert) von $U_{Cm}$.
$U_{Cm2}$ ist der Imaginärteil (Quadratur-Signalwert) von $U_{Cm}$.

$W_{R1m}$ wird als gemessener Wert des unten definierten $W_{R1}$ definiert.
$W_{R1m1}$ ist der Realteil (gleichphasiger Signalwert) von $W_{R1m}$.
$W_{R1m2}$ ist der Imaginärteil (Quadratur-Signalwert) von $W_{R1m}$.

$W_{R2m}$ wird als gemessener Wert des unten definierten $W_{R2}$ definiert.
$W_{R2m1}$ ist der Realteil (gleichphasiger Signalwert) von $W_{R2m}$.
$W_{R2m2}$ ist der Imaginärteil (Quadratur-Signalwert) von $W_{R2m}$.

$W_{Cm}$ wird als gemessener Wert des unten definierten $W_C$ definiert.
$W_{Cm1}$ ist der Realteil (gleichphasiger Signalwert) von $W_{Cm}$.
$W_{Cm2}$ ist der Imaginärteil (Quadratur-Signalwert) von $W_{Cm}$.

$U_C$ ist als komplexer Spannungswert definiert, der den die Zelle 13 durchfließenden Strom repräsentiert.
$U_{R1}$ ist als komplexer Spannungswert definiert, der den die erste Referenz durchfließenden Strom repräsentiert.
$U_{R2}$ ist als komplexer Spannungswert definiert, der den die zweite Referenz durchfließenden Strom repräsentiert.

$W_C$ ist als komplexer Spannungswert definiert, der die an der Zelle 13 anliegende Spannung repräsentiert.
$W_{R1}$ ist als komplexer Spannungswert definiert, der die an der ersten Referenz anliegende Spannung repräsentiert.
$W_{R2}$ ist als komplexer Spannungswert definiert, der die an der zweiten Referenz anliegende Spannung repräsentiert.

$I_C$ ist der komplexe, durch die Zelle fließende Strom.
$I_{R1}$ ist der komplexe, durch die erste Referenz fließende Strom.
$I_{R2}$ ist der komplexe, durch die zweite Referenz fließende Strom.

$V_C$ ist die komplexe Spannung an der Zelle.
$V_{R1}$ ist die komplexe Spannung an der ersten Referenz.
$V_{R2}$ ist die komplexe Spannung an der zweiten Referenz.

$Y_C$ ist der komplexe Leitwert der Zelle.

$Y_{R1}$ ist der komplexe Leitwert der ersten Referenz.

$Y_{R2}$ ist der komplexe Leitwert der zweiten Referenz.

H ist die Übertragungsfunktion des Schaltkreises, der komplexe Strommessungen durchführt.

G ist die Übertragungsfunktion des Schaltkreises, der komplexe Spannungsmessungen durchführt.

$V_S$ ist die erzeugte Quellenspannung.

$A_C$ ist die Übertragungsfunktion der Zellenbetriebsspannung.

$A_R$ ist die Übertragungsfunktion der Referenzbetriebsspannung.

D ist die Übertragungsfunktion der Phasen- und Gewinnfehlanpassung zwischen den gemessenen reellen (phasengleichen) und gemessenen imaginären (Quadratur-) Komponenten des komplexen Stroms und der Spannung. Diese Fehlanpassung wird durch Fehler in den Schaltkreiselementen bedingt, die die Messung durchführen. D ist auch als "Mischertransformationsmatrix" bekannt. Es ist Aufgabe der vorliegenden Erfindung, den Wert von D zu messen und seinen Einfluss auszugleichen.

Im Schaltkreis 100 zur Messung des Leitwerts wird eine erzeugte Quellspannung 102 ($V_S$) selektiv an die Zelle 13 oder an eine einer Mehrzahl von Referenzen über eine zugeordnete Übertragungsfunktion angelegt, wie durch die Bezugszeichen 104, 106 und 108 dargestellt. Wenn $V_S$ an die Übertragungsfunktion $A_C$ 104 angelegt wird, wird eine Spannung $V_C$ erzeugt, die an den komplexen Leitwert der Zelle $Y_C$ 110 angelegt wird. Wenn analog die Spannung 102 ($V_S$) an eine erste Übertragungsfunktion $A_R$ 106 angelegt wird, wird die entstehende Spannung $V_{R1}$ an den komplexen Leitwert des ersten Referenzleitwerts $Y_{R1}$ 112 angelegt, und wenn das Signal 102 an die zweite Übertragungsfunktion $A_R$ 108 angelegt wird, wird die entstehende Spannung $V_{R2}$ an den zweiten komplexen Leitwert 114 ($Y_{R2}$) angelegt. Jeder der entstehenden Ströme wird in einem Addierer 116 summiert. Wenn nur ein Pfad ausgewählt wird, wird nur eines dieser Signale nicht Null sein. Der sich ergebende Strom ist dann $I_C$, wenn die Zelle ausgewählt wird, $I_{R1}$ wenn die erste Referenz ausgewählt wird, und $I_{R2}$ wenn die zweite Zelle ausgewählt wird. Der entstehende Strom fließt durch einen Schaltkreis mit einer Übertragungsfunktion H 120, wobei H eine Übertragungsfunktion zur Umsetzung komplexen Stroms in eine komplexe Spannung für Messzwecke ist. Die entstehende, durch den Knoten 121 gemessene Spannung repräsentiert den komplexen Strom durch entweder den Leitwert der Zelle 13 oder einen der Referenzleitwerte. Die reellen (phasengleichen) und imaginären (Quadratur-) Komponenten dieser Spannung werden festgestellt, indem die Spannung an den Unterschaltkreis angelegt wird, der aus den Blöcken 122, 123 und 124 besteht, wie in Figur 5 gezeigt. Auf diese Weise werden daher Spannungen $U_{cm1}$ und $U_{cm2}$ gemessen, die den komplexen Strom durch die Zelle repräsentieren. Durch das Auswählen je einer der Referenzen können auch Spannungen gemessen werden, die den komplexen Strom durch die erste Referenz oder die zweite Referenz repräsentieren.

Zusätzlich zum Messen von Spannungen, die die komplexen Stromwerte repräsentieren, werden entsprechend des Schaltkreises auch Spannungen berechnet, die die komplexen Spannungswerte repräsentieren. Die Spannungen von der Zelle, $V_C$, der ersten Referenz, $V_{R1}$, und der zweiten Referenz, $V_{R2}$, werden an einen Addierer 118 angelegt. Da nur eine der Referenzen oder die Zelle gleichzeitig ausgewählt werden, wird nur einer dieser Werte von Null verschieden sein. Das Ergebnis wird an eine Übertragungsfunktion 126 angelegt, die eine komplexe Spannung am Knoten 127 ergibt. Die reellen und imaginären (phasengleichen und Quadratur-) Komponenten dieser Spannung werden durch Anlegen der Spannung an den Unterschaltkreis festgestellt, der aus den Blöcken 128, 129 und 130 besteht, wie in Figur 5 dargestellt.

Auf diese Weise ermöglicht der in Figur 5 dargestellte Schaltkreis eine Bestimmung der Real- und Imaginärteile sowohl der Spannung als auch des Stroms, der bzw. die mit einem ausgewählten Leitwert verbunden ist oder sind. Dieser Leitwert ist entweder mit der Zelle 13 des Kornfeuchtigkeitssensors 12 oder mit einer der Referenzleitwerte des Kornfeuchtigkeitssensors verbunden.

Zur weiteren Erklärung liegen die folgenden mathematischen Beziehungen vor:

$$G = W/V$$

$$H = U/I$$

In jedem Fall sind die jeweiligen Übertragungsfunktionen als Verhältnis des Ausgangswerts der Funktion zum Eingangswert der Funktion definiert. Außerdem ist der Leitwert mathematisch definiert als:

$$Y = I/V = (U/H)/(W/G) = (U/W) \cdot (G/H).$$

Sind diese allgemeinen Beziehungen gegeben, ist der Leitwert einer Referenz definiert als:

$$Y_R = U_R/W_R \cdot G/H.$$

Außerdem werden die Leitwerte der leeren Zelle 13, $Y_{CE}$, und einer vollen Zelle, $Y_{CF}$, folgendermaßen berechnet:

$$Y_{CE} = U_{CE}/W_{CE} \cdot G/H.$$

$$Y_{CF} = U_{CF}/W_{CF} \cdot G/H.$$

Wenn die Messungen für den Referenzleitwert und den Leitwert der Zelle bei denselben Umgebungsbedingungen durchgeführt werden, kann angenommen werden, dass sowohl H als auch G in den Leitwertgleichungen für die Zelle und die Referenzen gleich sind. Dann kennzeichnet das Folgende die Kalibrierfaktoren für die leere Zelle und die Referenzen:

$$W_R/U_R \cdot Y_R = W_{CE}/U_{CE} \cdot Y_{CE} => F = W_{CE}/U_{CE} \cdot U_R/W_R = Y_R/Y_{CE}$$

Der Kalibrierfaktor F der Referenz gibt das Verhältnis vom Leitwert der Referenz zum Leitwert der leeren Zelle bei denselben Umgebungsbedingungen wieder. Daher kann zu Kalibrierzwecken ein Referenzleitwert anstelle eines Leitwerts der leeren Zelle verwendet werden.

Unter der Annahme, dass F konstant bleiben wird, kann die komplexe Dielektrizitätskonstante der Kornprobe berechnet werden zu:

$$\varepsilon = (U_{CF} \cdot W_R)/(W_{CF} \cdot U_R) \cdot F,$$

wobei

$$\varepsilon = \varepsilon' - j \varepsilon''.$$

Daher ermöglicht die vorliegende Erfindung eine Messung des komplexen Leitwerts des Korns zu Feuchtigkeitsmesszwecken.

Um genaue Strom- und Spannungsmessungen durchzuführen, ist es zweckmäßig, dass die phasengleichen (IP) und Quadratur- (Q) Signale der lokalen Oszillatoren, die bei den Mischern 216, 220 und 224 zum Extrahieren der reellen und imaginären Komponenten komplexer Signale verwendet werden, eine Phasendifferenz von genau 90 Grad und an ihren Grundfrequenzen identische Amplituden haben. Fehler werden in dem Maß eingeführt, in dem das nicht der Fall ist. Durch die Verwendung zweier Referenzleitwerte mit bekannten und stabilen Werten werden jedoch Korrekturen dieser Fehler durchgeführt.

Die D-Funktionen 124 und 130 repräsentieren die Verzerrung des Imaginärteils gegenüber dem Realteil aller gemessener komplexer Werte. Alle gemessenen Werte $U_m$ und $W_m$ können durch Verwendung derselben Formel korrigiert werden, um U und W zu erhalten, welches die Werte sind, bevor jegliche Messwertverzerrfehler eingeführt werden.

Folgendes ist die verzerrte Beziehung zwischen den komplexen Spannungen, die die Ströme durch die Zelle und die Referenzen und ihre gemessenen Werte repräsentieren:

$$U = [1 \; j] \cdot D^{-1} \cdot U_m,$$

wobei

$$D^{-1} = PFC = \begin{bmatrix} 1 & 0 \\ Pfc1 & pfc2 \end{bmatrix}$$

$$U_m = \begin{pmatrix} U_{m1} \\ U_{m2} \end{pmatrix}$$

Dieselbe verzerrte Beziehung gilt zwischen den komplexen Spannungen, die die Zell- und Referenzspannungen und ihre gemessenen Werte repräsentieren:

$$W = [1 \ j] \cdot D^{-1} \cdot W_m,$$

wobei

$$D^{-1} = PFC = \begin{bmatrix} 1 & 0 \\ Pfc1 & pfc2 \end{bmatrix}$$

$$W_m = \begin{pmatrix} W_{m1} \\ W_{m2} \end{pmatrix}$$

Das Expandieren der obigen Gleichungen ergibt:

$$U = U_{m1} + j \cdot (pfc1 \cdot U_{m1} + pfc2 \cdot U_{m2})$$

$$W = W_{m1} + j \cdot (pfc1 \cdot W_{m1} + pfc2 \cdot W_{m2})$$

Die Korrekturfaktoren pfc1 und pfc2 werden durch die Verwendung zweier unterschiedlicher Referenzen herausgefunden, die bekannte und stabile Leitwerte mit verschiedenen Phasenwinkeln haben. Als Beispiel ist in einer Ausführungsform der Erfindung der erste Referenzwert ein temperaturstabiler Kondensator mit 1 % Toleranz (COG) mit einem Wert von 15 pF (Leitwert $Y_{R1}$) und die zweite Referenz ein Präzisionswiderstand mit 0,1 % Toleranz mit einem Wert von 2000 $\Omega$ (Leitwert $Y_{R2}$). Auch andere Referenzwerte können genutzt werden.

Das Verhältnis der Referenzleitwerte wird folgendermaßen berechnet:

$$R = Y_{R1}/Y_{R2} = Q_R + j \cdot Q_I.$$

Das Verhältnis der rohen Messwerte der zwei Referenzen ist:

$$R_m = (U_{R1} \cdot W_{R2}) / (W_{R1} \cdot U_{R2}).$$

In die obige Gleichung können die Ergebnisse der Messungen eingefügt werden, so dass man erhält:

$$R_m = ((U_{R1m1}+j(U_{R1m1} \cdot pfc1+U_{R1m2} \cdot pfc2))(W_{R2m1}+j(W_{R2m1} \cdot pfc1+W_{R2m2} \cdot pfc2)))$$
$$/ ((W_{R1m1}+j(W_{R1m1} \cdot pfc1+W_{R1m2} \cdot pfc2))(U_{R2m1}+j(U_{R2m1} \cdot pfc1+U_{R2m2} \cdot pfc2))).$$

$R_m$ wird mit R gleichgesetzt und zwei quadratische Gleichungen mit zwei Unbekannten (pfc1, pfc2) werden abgeleitet:

$$a_1 \cdot pfc1^2 + b_1 \cdot pfc2^2 + c_1 \cdot pcf1 \cdot pfc2 + d_1 \cdot pfc1 + e_1 \cdot pfc2 + f_1 = 0$$

(vom Realteil)

$$a_2 \cdot pfc1^2 + b_2 \cdot pfc2^2 + c_2 \cdot pcf1 \cdot pfc2 + d_2 \cdot pfc1 + e_2 \cdot pfc2 + f_2 = 0$$

(vom Imaginärteil), wobei

$$a_1 = Q_R \ W_{R1m1} \cdot U_{R2m1} \ - \ U_{R1m1} \cdot W_{R2m1}$$

$$a_2 = Q_I \ W_{R1m1} \cdot U_{R2m1}$$

$$b_1 = Q_R \ W_{R1m2} \cdot U_{R2m2} \ - \ U_{R1m2} \cdot W_{R2m2}$$

$$b_2 = Q_I \ W_{R1m2} \cdot U_{R2m21}$$

$$c_1 = Q_R \ W_{R1m1} \cdot U_{R2m2} \ + \ Q_R \cdot \ W_{R1m2} \cdot U_{R2m1} \ - \ U_{R1m1} \cdot W_{R2m2} \ - \ U_{R1m2} \cdot W_{R2m1C}$$

$$c_2 = e_1$$

$$d_1 = 2 \ \cdot \ Q_I \ W_{R1m1} \cdot U_{R2m1}$$

$$d_2 = -2 \ \cdot \ a_1$$

$$e_1 = Q_I \ \cdot \ ( \ W_{R1m1} \cdot U_{R2m2} \ + \ W_{R1m2} \cdot U_{R2m1)}$$

$$e_2 = - \ c_1$$

$$f_1 = - \ a_1$$

$$f_2 = - a_2$$

Diese zwei quadratischen Gleichungen werden dann gleichzeitig für pfc1 und pfc2 gelöst. Da eine einfache, geschlossene Form nicht vorhanden ist, können sie beispielsweise durch Newton-Raphson-Iteration gelöst werden. Andere Algorithmen zur Lösung numerischer Gleichungen können ebenfalls verwendet werden. Es ist bekannt, dass die Lösung nahe des Punktes (pcf1=0, pfc2=1) liegt, so dass diese vorzugsweise als Startpunkt verwendet werden. Theoretisch sind vier verschiedene Lösungen möglich. Jede Lösung, die nicht nahe (0,1) liegt, sollte als ungeeignet angesehen werden. Bei einer Softwareimplementation kann eine geeignete Fehlerbedingung gesetzt werden. Es ist nicht wahrscheinlich, dass dies vorkommt, jedoch können Vorsichtsmaßnahmen für den Fall, dass es vorkommt, vorgesehen werden.

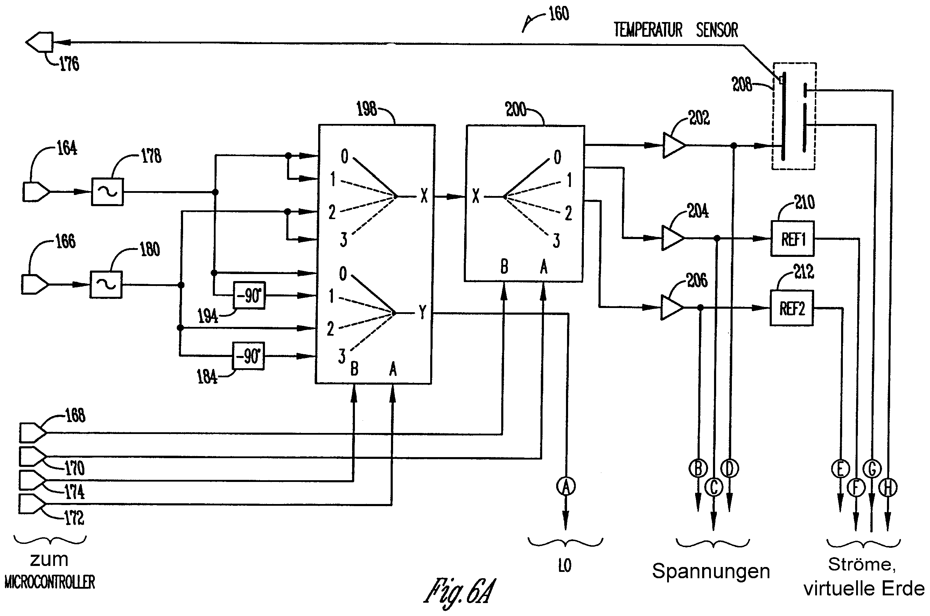

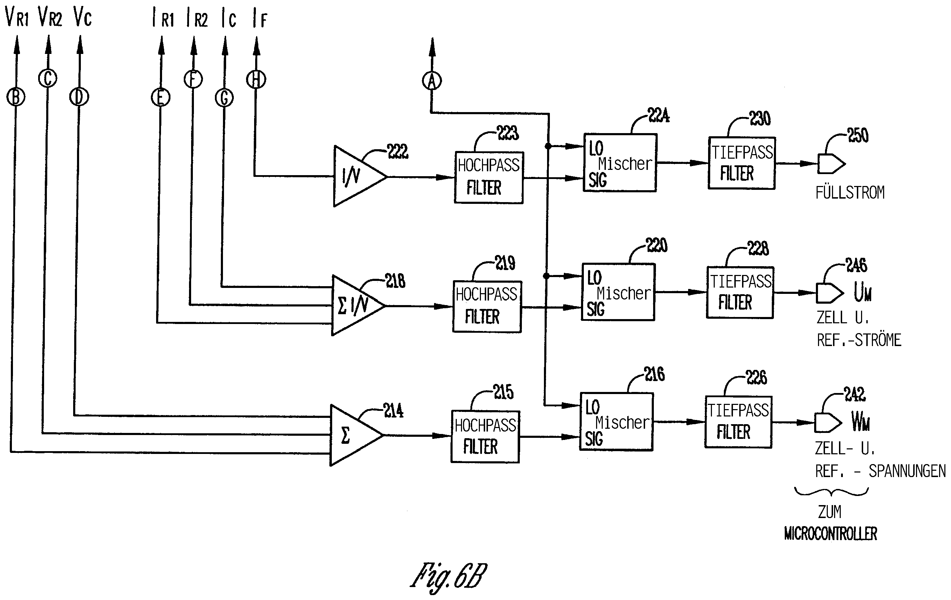

Die Figuren 6A und 6B zeigen ein Schema für den erfindungsgemäßen Kornfeuchtigkeitssensor. Das Schema zeigt eine Anzahl an Eingangs- und Ausgangsleitungen zur Verbindung mit einer intelligenten Steuerung, wie einem Prozessor, Mikrocontroller, integriertem Schaltkreis oder anderem Gerät. Dieses Schema zeigt nur eine Schaltkreiskonfiguration gemäß der vorliegenden Erfindung. Die vorliegende Erfindung stellt die Möglichkeit bereit, selektiv eine von einer Mehrzahl komplexer Leitwerte bei einer Vielzahl von Frequenzen zu messen.

Die Eingänge in das System (Ausgänge einer intelligenten Steuerung) werden in Figur 6A gezeigt. Die Eingänge umfassen einen ersten Frequenzeingang 164 und einen zweiten Frequenzeingang 166. Optional werden ein erster Sinuswellengenerator 178 und ein zweiter Sinuswellengenerator 180 verwendet. Die Sinuswellengeneratoren nehmen den Rechteckwellenausgang eines Mikrocontrollers, teilen die Frequenz wie erforderlich und glätten den Ausgang, so dass ein sinusförmiges Signal erzeugt wird. Der Ausgang des ersten Sinuswellengenerators 178 ist elektrisch mit drei Schaltereingängen eines doppelten Umschalters 198 mit vier Eingängen verbunden. Zusätzlich ist der Ausgang des ersten Sinuswellengenerators 178 elektrisch mit einem 90° Phasenschieber 194 verbunden. Der 90° Phasenschieber 194 ist derart aufgebaut, dass sein Ausgangssignal um 90° in der Phase gegenüber seinem Eingangssignal verschoben ist. Der 90° Phasenschieber ist elektrisch mit dem Schaltereingang des doppelten Umschalters 198 verbunden. Der Ausgang des zweiten Sinuswellengenerators 180 ist ähnlich angeschlossen.

Der erste Sinuswellengenerator 178 und der zweite Sinuswellengenerator 180 arbeiten bei unterschiedlichen Frequenzen. Beispielsweise arbeitet der erste Sinuswellengenerator 178 bei 10 MHz, während der zweite Sinuswellengenerator bei 1 MHz arbeitet.

Der doppelte Umschalter 198 wird durch die Eingänge 172 und 174 gesteuert, die verwendet werden, eines der Signale auszuwählen. Einer der Ausgänge des Schalters 198 ist elektrisch mit einem Eingang des doppelten Umschalters 200 mit zwei Ausgängen verbunden. Eingänge 168 und 170 sind mit dem Umschalter 200 verbunden, um zu steuern, welcher der Ausgänge ausgewählt wird. Die Ausgänge sind gepuffert und dann elektrisch mit der Sensorzelle 208 (die der Zelle 13 in den anderen Figuren entspricht), einem ersten Referenzleitwert 210 und einem zweiten Referenzleitwert 212 verbunden. Die Referenzleitwerte werden für Kalibrierzwecke verwendet.

Wie in Figur 6B gezeigt, sind die gepufferten Ausgänge, die die Zelle 208 und die beiden Referenzen treiben, auch elektrisch mit einem Summierkreis 214 verbunden. Der Ausgang des Summierkreises ist elektrisch über einen Hochpassfilter 215 mit einem Mischer 216 verbunden. Der Mischer 216 hat auch einen lokalen Oszillatoreingang, der elektrisch mit einem Ausgang des Schalters 198 (Figur 6A) verbunden ist. Der Ausgang des Mischers 216 geht durch einen Tiefpassfilter 226 und ist dann elektrisch mit einem Analog/Digitalwandler verbunden und wird durch den Mikrocontroller ausgelesen. Am Ausgang des Mischers 216 liegt eine Gleichspannung an, die proportional zur Komponente des Eingangsspannungssignals ist, welche mit dem lokalen Oszillator gleichphasig ist.

Die Sensorplatte 68 der Zelle 208 und die erste Referenz 210 und die zweite Referenz 212 aus Figur 6A sind elektrisch mit einem summierenden Strom/Spannungsumsetzer 218 verbunden, wie er in Figur 6B gezeigt ist. Der summierende Strom/Spannungsumsetzer 218 hat einen Eingang mit niedriger Impedanz, der virtuell auf Erdpotential liegt. Der Ausgang des summierenden Strom/Spannungsumsetzers 218 ist über ein Hochpassfilter 219 elektrisch mit einem zweiten Mischer 220 verbunden. Der zweite Mischer 220 hat auch einen lokalen Oszillatoreingang, der elektrisch mit einem Ausgang des Schalters 198 (Figur 6A) verbunden ist. Der Ausgang des Mischers 220 geht durch einen Tiefpassfilter 228 und ist dann elektrisch mit einem Analog/Digitalwandler verbunden und wird durch den Mikrocontroller ausgelesen. Am Ausgang des Mischers 220 liegt eine Gleichspannung an, die proportional zur Komponente des Eingangssignals ist, welches mit dem lokalen Oszillator gleichphasig ist.

Außerdem läuft der Strom $I_F$ von der Füllstandssensorplatte 66 an der Zelle 208 (s. Figur 6A) durch den Strom/Spannungsumsetzer 222. Dieser Strom/Spannungsumsetzer 222 hat ebenfalls einen Eingang mit niedriger Impedanz, der sich virtuell auf Erdpotential befindet. Der Ausgang des Strom/Spannungsumsetzers 222 ist über ein Hochpassfilter 223 mit einem dritten Mischer 224 verbunden. Der dritte Mischer 224 hat auch einen lokalen Oszillatoreingang, der elektrisch mit einem Ausgang des Schalters 198 verbunden ist. Der Ausgang des Mischers 224 geht über einen Tiefpassfilter 230 an einen Analog/Digitalwandler und wird vom Mikrocontroller ausgelesen. Diese Konfiguration ermöglicht eine Überwachung des Leitwerts der Füllstandssensorplatte 66 gegenüber der Sensorplatte 68. Wenn dieses Verhältnis proportional zu den relativen Größen der Platten 66, 68 ist, dann wird die Zelle 208 als mit Korn gefüllt angesehen.

Das synchrone Nachweisverfahren der Messung komplexer Signale unter Verwendung eines lokalen Oszillators, eines Mischers und eines Tiefpassfilters, wie es oben beschrieben wurde, hat einen sehr engen Bandpassfiltereffekt, der den Untergrundgeräuscheinfluss auf die Messung wesentlich reduziert. Das Verfahren der Messung sehr kleiner Ströme mit virtueller Erde wird genutzt, um den Vorteil einer wesentlichen Verminderung des Einflusses parasitärer Elemente auf den Stromsummier- und Messknoten zu erzielen.

In der Figur 6A ist ein Thermistor oder ein anderer Temperatursensor an der elektrisch beaufschlagten Platte 64 der Zelle 208 angebracht. Dies ist nur ein Beispiel der Anbringung eines Temperatursensors. Der Temperatursensor kann auch an einer der anderen Platten der Zelle 208 angebracht sein. Die Messung der Temperatur erlaubt eine entsprechende Korrektur der Feuchtigkeitsberechnungen.

**Patentansprüche**

1. Kornfeuchtigkeitssensor für einen Mähdrescher, mit einem Schaltkreis, der eine Spannungsquelle (178, 180) und mehrere Referenzen (210, 212) umfasst, einer mit der Spannungsquelle (178, 180) verbindbaren Platte (64) und einer Sensorplatte (68), die in der Nähe der mit der Spannungsquelle (178, 180) verbindbaren Platte (64) angeordnet und zu ihr parallel orientiert ist, um im Zwischenraum zwischen den Platten (64, 68) die Kapazität zu messen, wobei die Spannungsquelle (178, 180) eingerichtet ist, unterschiedliche Frequenzen an die mit ihr verbindbare Platte (64) anzulegen, der Schaltkreis eingerichtet ist, den komplexen Leitwert zwischen der mit der Spannungsquelle (178, 180) verbindbaren Platte (64) und der Sensorplatte (68) mit dazwischen eingefülltem Korn zu messen und der Schaltkreis eingerichtet ist, eine Referenz (210, 212) mit der Spannungsquelle (178, 180) zu verbinden und den komplexen Leitwert der Referenz (210, 212) zu messen um eine komplexe Dielektrizitätskonstante aus den beiden gemessenen, komplexen Leitwerten zu errechnen, **dadurch gekennzeichnet, dass** die Referenzen (210, 212) von den Platten (64, 68) getrennt sind, dass der Schaltkreis eingerichtet ist Messungen für den Leitwert einer der Referenzen (210, 212) und den Leitwert der leeren Zelle (13, 208) bei denselben Umgebungsbedingungen durchzuführen, um zu Kalibrierzwecken einen Referenzleitwert anstelle eines Leitwerts der leeren Zelle (13, 208) zu verwenden, wobei der Referenzleitwert über einen Kalibrierfaktor F, der das Verhältnis von Leitwert der Referenz (210, 212) zum Leitwert der leeren Zelle (13, 208) wiedergibt, bestimmt wird, und dass der Schaltkreis eingerichtet ist, unter mehreren Referenzen (210, 212) auszuwählen.

2. Kornfeuchtigkeitssensor nach Anspruch 1, **gekennzeichnet durch** eine Abschirmung (70), die im Abstand von der Sensorplatte (68) und parallel dazu auf der von der mit der Spannungsquelle (178, 180) verbindbaren Platte (64) abgewandten Seite der Sensorplatte (68) angeordnet ist.

3. Kornfeuchtigkeitssensor nach Anspruch 2, **dadurch gekennzeichnet, dass** die Abschirmung (70) mit demselben elektrischen Potential verbunden ist wie die Sensorplatte (68) .

4. Kornfeuchtigkeitssensor nach einem der Ansprüche 1 bis 3, **dadurch gekennzeichnet, dass** ein Temperatursensor mit einer die Platten (64, 66, 68) enthaltenden Zelle (13, 208) gekoppelt ist, um eine etwa der Korntemperatur entsprechende Temperatur zu erfassen.

5. Kornfeuchtigkeitssensor nach einem der Ansprüche 1 bis 4, **dadurch gekennzeichnet, dass** der Schaltkreis eingerichtet ist, eine Mehrzahl auswählbarer Signaleingänge mit der mit der Spannungsquelle (178, 180) verbindbaren Platte (64) zu koppeln, die jeweils mit einer zugeordneten Frequenz arbeiten.

6. Kornfeuchtigkeitssensor nach einem der Ansprüche 1 bis 5, **dadurch gekennzeichnet, dass** der Schaltkreis eingerichtet ist, die Real- und Imaginärkomponenten einer an die mit der Spannungsquelle (178, 180) verbindbaren Platte (64) angelegten Spannung zu messen, dass der Schaltkreis eingerichtet ist, die Real- und Imaginärkomponenten eines an der Sensorplatte (68) fließenden Stroms zu messen.

7. Kornfeuchtigkeitssensor nach Anspruch 6, **dadurch gekennzeichnet, dass** der Schaltkreis eingerichtet ist, die Spannung und/oder der Strom durch einen Synchrondetektor, insbesondere Mischer (216, 220), zu messen.

8. Kornfeuchtigkeitssensor nach Anspruch 7, **dadurch gekennzeichnet, dass** der Synchrondetektor zwischen einer Messung des Realteils und des Imaginärteils der Spannung bzw. des Stroms umschaltbar ist.

9. Kornfeuchtigkeitssensor nach einem der Ansprüche 1 bis 8, **dadurch gekennzeichnet, dass** eine Füllstandssensorplatte (66) in der Nähe der Sensorplatte (68) und parallel zur mit der Spannungsquelle (178, 180) verbindbaren Platte (64) angeordnet ist, um zu erfassen, ob der Zwischenraum mit Korn gefüllt ist.

**Claims**

1. Grain moisture sensor for a combine harvester, having a circuit, which comprises a voltage source (178, 180) and a plurality of references (210, 212), having a plate (64) that can be connected to the voltage source (178, 180) and having a sensor plate (68), which is arranged near the plate (64) that can be connected to the voltage source (178, 180) and which is aligned in parallel therewith, in order to measure the capacitance in the intermediate space between the plates (64, 68), wherein the voltage source (178, 180) is configured to apply different frequencies to the plate (64) that can be connected to said voltage source, the circuit is configured to measure the complex conductance between the plate (64) that can be connected to the voltage source (178, 180) and the sensor plate (68) with grain poured in between said plates, and the circuit is configured to connect a reference (210, 212) to the voltage source (178, 180) and to measure the complex conductance of the reference (210, 212) in order to calculate a complex dielectric constant from the two measured complex conductances, **characterized in that** the references (210, 212) are isolated from the plates (64, 68), **in that** the circuit is configured to carry out measurements for the conductance of one of the references (210, 212) and the conductance of the empty cell (13, 208) in the same ambient conditions in order to use a reference conductance instead of a conductance of the empty cell (13, 208) for calibration purposes, wherein the reference conductance is determined by means of a calibration factor F, which describes the ratio of the conductance of the reference (210, 212) to the conductance of the empty cell (13, 208), and **in that** the circuit is configured to make a selection from a plurality of references (210, 212).

2. Grain moisture sensor according to Claim 1, **characterized by** a shielding (70), which is arranged at a distance from the sensor plate (68) and in parallel therewith on the side of the sensor plate (68) that faces away from the plate (64) that can be connected to the voltage source (178, 180).

3. Grain moisture sensor according to Claim 2, **characterized in that** the shielding (70) is connected to the same electrical potential as the sensor plate (68).

4. Grain moisture sensor according to one of Claims 1 to 3, **characterized in that** a temperature sensor is coupled to a cell (13, 208) containing the plates (64, 66, 68) in order to detect a temperature that corresponds approximately to the grain temperature.

5. Grain moisture sensor according to one of Claims 1 to 4, **characterized in that** the circuit is configured to couple a plurality of selectable signal inputs to the plate (64) that can be connected to the voltage source (178, 180), said signal inputs each operating at an assigned frequency.

6. Grain moisture sensor according to one of Claims 1 to 5, **characterized in that** the circuit is configured to measure the real and imaginary components of a voltage applied to the plate (64) that can be connected to the voltage source (178, 180), **in that** the circuit is configured to measure the real and imaginary components of a current flowing at the sensor plate (68).

7. Grain moisture sensor according to Claim 6, **characterized in that** the circuit is configured to measure the voltage and/or the current by way of a synchronous detector, in particular a mixer (216, 220).

8. Grain moisture sensor according to Claim 7, **characterized in that** the synchronous detector can be switched between a measurement of the real part and the imaginary part of the voltage and of the current, respectively.

9. Grain moisture sensor according to one of Claims 1 to 8, **characterized in that** a fill level sensor plate (66) is arranged near the sensor plate (68) and in parallel with the plate (64) that can be connected to the voltage source (178, 180) in order to detect whether the intermediate space has been filled with grain.

**Revendications**

1. Capteur d'humidité de grain pour moissonneuse-batteuse, comportant un circuit qui comprend une source de tension (178, 180) et une pluralité de références (210, 212), une plaque (64) pouvant être connectée à la source de tension (178, 180), et une plaque de capteur (68) disposée à proximité de la plaque (64) pouvant être connectée à la source de tension (178, 180) et orientée parallèlement à celle-ci, afin de mesurer la capacité dans l'espace séparant les plaques (64, 68), dans lequel la source de tension (178, 180) est conçue pour appliquer différentes fréquences à la plaque (64) qui peut lui être connectée, le circuit est conçu pour mesurer la conductance complexe entre la plaque (64) pouvant être connectée à la source de tension (178, 180) et la plaque de capteur (68) en présence de grain remplissant l'espace les séparant et le circuit est conçu pour connecter une référence (210, 212) à la source de tension (178, 180) et pour mesurer la conductance complexe de la référence (210, 212), afin de calculer une constante diélectrique complexe à partir des deux conductances complexes mesurées, **caractérisé en ce que** les références (210, 212) sont séparées des plaques (64, 68), **en ce que** le circuit est conçu pour effectuer des mesures de la conductance de l'une des références (210, 212) et de la conductance de la cellule vide (13, 208) dans les mêmes conditions ambiantes, afin d'utiliser une conductance de référence au lieu d'une conductance de la cellule vide (13, 208) à des fins d'étalonnage, dans lequel la conductance de référence est déterminée par l'intermédiaire d'un facteur d'étalonnage F représentant le rapport de la conductance de la référence (210, 212) à la conductance de la cellule vide (13, 208), et **en ce que** le circuit est conçu pour effectuer une sélection parmi une pluralité de références (210, 212).

2. Capteur d'humidité de grain selon la revendication 1, **caractérisé par** un blindage (70) qui est disposé à distance de la plaque de capteur (68) et parallèlement à celle-ci sur le côté de la plaque de capteur (68) qui est tourné à l'opposé de la plaque (64) pouvant être connectée à la source de tension (178, 180).

3. Capteur d'humidité de grain selon la revendication 2, **caractérisé en ce que** le blindage (70) est connecté au même potentiel électrique que la plaque de capteur (68).

4. Capteur d'humidité de grain selon l'une des revendications 1 à 3, **caractérisé en ce qu'**un capteur de température est couplé à une cellule (13, 208) contenant les plaques (64, 66, 68), afin de détecter une température correspondant approximativement à la température du grain.

5. Capteur d'humidité de grain selon l'une des revendications 1 à 4, **caractérisé en ce que** le circuit est conçu pour coupler une pluralité d'entrées de signaux sélectionnables à la plaque (64) pouvant être connectée à la source de tension (178, 180), chacune de ces entrées fonctionnant à une fréquence associée.

6. Capteur d'humidité de grain selon l'une des revendications 1 à 5, **caractérisé en ce que** le circuit est conçu pour mesurer les composantes réelle et imaginaire d'une tension appliquée à la plaque (64) pouvant être connectée à la source de tension (178, 180), **en ce que** le circuit est conçu pour mesurer les composantes réelle et imaginaire d'un courant passant sur la plaque de capteur (68).

7. Capteur d'humidité de grain selon la revendication 6, **caractérisé en ce que** le circuit est conçu pour mesurer la tension et/ou le courant au moyen d'un détecteur synchrone, en particulier un mélangeur (216, 220).

8. Capteur d'humidité de grain selon la revendication 7, **caractérisé en ce que** le détecteur synchrone peut être amené à basculer entre une mesure de la partie réelle et une mesure de la partie imaginaire de la tension ou du courant.

9. Capteur d'humidité de grain selon l'une des revendications 1 à 8, **caractérisé en ce qu'**une plaque de capteur de niveau (66) est disposée à proximité de la plaque de capteur (68) et parallèlement à la plaque (64) pouvant être connectée à la source de tension (178, 180), afin de détecter si ledit espace est rempli de grain.

Fig. 1A

*Fig.1B*

*Fig. 1C*

*Fig.2*

*Fig.3*

Fig.4

Fig.5

19

*Fig.6A*

*Fig.6B*

EP 1 811 293 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- GB 2210693 A

- US 4399404 A

**In der Beschreibung aufgeführte Nicht-Patentliteratur**

- **K. C. LAWRENCE et al.** Wheat Moisture Determination by 1- to 110-MHz Swept-Frequency Admittance Measurements. *Transactions of the ASAE,* Februar 1998, vol. 41 (1), 135-142